# EUROPEAN PATENT SPECIFICATION

(11) **EP 4 422 554 B1**
(45) Date of publication and mention of the grant of the patent: **30.09.2026**
(21) Application number: 22884823.0
(22) Date of filing: 25.10.2022
(51) Int. Cl.: A61F 2/28, A61B 17/80, A61B 17/68

(54) **CRANIOPLASTY PROSTHESIS AND COMPONENTS THEREFOR**
KRANIOPLASTIEPROTHESE UND KOMPONENTEN DAFÜR
PROTHÈSE DE CRANIOPLASTIE ET COMPOSANTS ASSOCIÉS

(30) Priority: 25.10.2021 US 202163271432 P
(43) Date of publication of application: 04.09.2024
(73) Proprietor: Société de Commercialisation des Produits de la Recherche Appliquée SOCPRA Sciences Santé et Humaines S.E.C., Sherbrooke, Québec J1L 1E2 (CA)
(72) Inventor: LAPOINTE, Simon, Sherbrooke, Québec J1L 3A2 (CA); IORIO-MORIN, Christian, Sherbrooke, Québec J1N 3W5 (CA); TOUCHETTE, Charles, Sherbrooke, Québec J1G 0B5 (CA); BERGERON, Serge, Québec J0B 1C0 (CA)
(74) Representative: Plasseraud IP
(86) International application number: PCT/CA2022/051571
(87) International publication number: WO 2023/070200

(56) References cited:
- CN-A- 110 801 314
- CN-A- 113 456 290
- US-A1- 2012 203 284
- US-A1- 2015 105 806
- US-A1- 2015 374 497
- US-A1- 2020 197 046

## Description

### CROSS-REFERENCE TO RELATED APPLICATION

The present application claims the priority of United States Patent Application No. 63/271,432, filed on October 25, 2021

### TECHNICAL FIELD

The present application pertains to implants, attachment devices, fixation plates, plating systems, and/or prostheses used in surgical procedures such as craniotomies, craniectomies and/or cranioplasties.

### BACKGROUND

Some neurosurgical procedures pertain to the temporary or permanent removal of a bone flap from a skull for various reasons. For example, a craniotomy is a procedure by which a bone flap is temporarily removed to access part of a brain, blood vessels or like soft tissue within the skull. In a craniotomy, the bone flap is replaced at the end of the procedure, before skin closure. A craniectomy is a procedure by which the bone flap is permanently removed from a remainder of the skull. This can be performed to reduce intracranial pressure (decompressive craniectomy), because the skull itself is fractured beyond repair, or infected and must be removed for the infection to heal. In a craniectomy, the skin is closed without the bone flap in place, leaving an area of the brain unprotected. A cranioplasty is the reinsertion of the bone flap or equivalent prosthesis to cover the opening in the skull.

Cranioplasty may often occur several weeks after a craniectomy, once the intracranial pressure has returned to acceptable levels or the infection has cleared. In some instances, custom-made cranial prostheses are used in cranioplasty, post-craniectomy. As it is difficult to predict the geometrical parameters of a bone flap prior to surgery, there may result an extended period in which a skull has an uncovered craniectomy opening, leaving a portion of the brain unprotected structurally at the skull. In the case of decompressive craniectomy, to allow a reduction in the intracranial pressure, there must often be a period in which the bone flap is removed.

Therefore, patients in the wait for cranioplasty may be at high risk of injury and must often be kept in a hospital or like controlled environment. As one possible occurrence, the sinking flap syndrome may occur and may involve serious complications, such as paralysis or coma. Moreover, a subsequent intervention is then required to install a cranial prosthesis or bone flap, resulting in costs and further hospitalization time.

Dynamic systems have been developed to allow bone flaps to move and expand the intracranial volume, thereby allowing a "decompressive craniotomy". The dynamic systems may typically include telescopic or spring-based expansion. However, such systems may be voluminous and may exhibit unnecessary resistance to bone flap or prosthesis movement, thereby providing insufficient decompression. Moreover, dynamic systems voluminous in nature may be unesthetic in that they may create lumps on one's head.

Document US 2012/203284 A1 can be considered as the closest prior art to the invention as defined in the appended claims.

### SUMMARY

In a first aspect, there is provided an attachment device for cranioplasty comprising a body defined from a sheet material and including: a first connection end having at least a first connection hole configured to cooperate with a fastener to anchor the attachment device to a bone flap or prosthesis covering at least part of the opening in the skull, a second connection end having at least a second connection hole configured to cooperate with a fastener to anchor the attachment device to a skull adjacent to an opening in the skull, a frame portion extending from the first connection end, and a coil portion between the frame portion and the second connection end, the coil portion having struts configured for deforming in flexion, and webs between the struts configured for deforming in torsion, wherein the frame portion is configured to be located over a periphery of the opening in the skull to block inward movement, and wherein the coil portion enables an out-of-plane deformation of the attachment device for the first connection end to move out of a neutral plane with the second connection end.

Further in accordance with the first aspect, for example, the body extends lengthwise from the first connection end to the second connection end, with at least some of the struts extending at least partially lengthwise.

Still further in accordance with the first aspect, for example, at least some of the webs extending at least partially widthwise.

Still further in accordance with the first aspect, for example, the second connection end has a pair of the second connection holes.

Still further in accordance with the first aspect, for example, the coil portion has a first set of coils and a second set of coils, the first set of coils and the second set of coils connected to a respective one of the second connection holes and merging at the frame portion.

Still further in accordance with the first aspect, for example, the second connection holes form rotational joints with fasteners configured to secured the second connection end to the skull, the rotational joints enabling lengthwise expansion of the attachment device.

Still further in accordance with the first aspect, for example, a part of the coil portion is on one side of a line passing through the second connection holes, the first connection end being on the other side of the line.

Still further in accordance with the first aspect, for example, the part flares in toward the line.

Still further in accordance with the first aspect, for example, the frame portion has a closed frame between the first connection end and the coil portion.

Still further in accordance with the first aspect, for example, an entirety of the contour edges of the body is arcuate.

Still further in accordance with the first aspect, for example, the first connection end has a pair of the first connection holes.

Still further in accordance with the first aspect, for example, the frame portion has a strut extending lengthwise from the coil portion.

Still further in accordance with the first aspect, for example, the attachment device has a symmetry axis extending lengthwise.

In accordance with a second aspect, there is provided a cranioplasty prosthesis comprising: a pair of the attachment device as described above; and a prosthetic blade a body defined from a sheet material and including: a first connection end configured to be anchored to a first position of a skull by one of the attachment devices, a second connection end configured to be anchored to a second position of the skull by the other of the attachment devices, the first position and the second position being separated by a skull opening, and an elongated plate portion between the first connection end and the second connection end, the elongated plate portion having a main surface facing toward the skull opening, wherein the sheet material is a rigid biocompatible material deformable out of plane.

Further in accordance with the second aspect, for example, the body has holes.

Still further in accordance with the second aspect, for example, the body is elongated.

Still further in accordance with the second aspect, for example, the attachment devices and the prosthetic blade are a monoblock component.

In accordance with a third aspect, there is provided a prosthetic blade for covering a skull opening in cranioplasty comprising: a body defined from a sheet material and including: a first connection end configured to be anchored to a first position of a skull, a second connection end configured to be anchored to a second position of the skull, the first position and the second position being separated by the skull opening, and an elongated plate portion between the first connection end and the second connection end, the elongated plate portion having a main surface facing toward the skull opening; wherein the sheet material is a rigid biocompatible material deformable out of plane.

Further in accordance with the third aspect, for example, the body has holes.

Still further in accordance with the third aspect, for example, the body is elongated.

### DESCRIPTION OF THE DRAWINGS

Reference is now made to the accompanying figures in which:
Fig. 1 is a perspective view of a cranioplasty prosthesis relative to an opening in a skull in accordance with an embodiment of the present disclosure;
Fig. 2 is an enlarged view of an end of the cranioplasty prosthesis of Fig. 1;
Fig. 3 is a perspective view of the cranioplasty prosthesis of Fig. 1;
Fig. 4 is a plane view of a series of prosthetic blades relative to an opening in a skull in accordance with another embodiment of the present disclosure;
Fig. 5 is a plane view of an attachment device in accordance with another embodiment of the present disclosure;
Fig. 6 is a perspective view showing an out-of-plane deformation and a neutral position of the attachment device of Fig. 5;
Fig. 7 is an elevation view of the attachment device of Fig. 5 in the out-of-plane deformation;
Fig. 8 is a series of plane views showing contemplated shapes for the attachment device of the present disclosure;
Fig. 9 is a graph showing a displacement to force relation of the attachment device of Fig. 5 relative to an attachment device operating according to Hooke's law; and
Fig. 10 is a plan view of an attachment device in accordance with a variant of the present disclosure.

### DETAILED DESCRIPTION

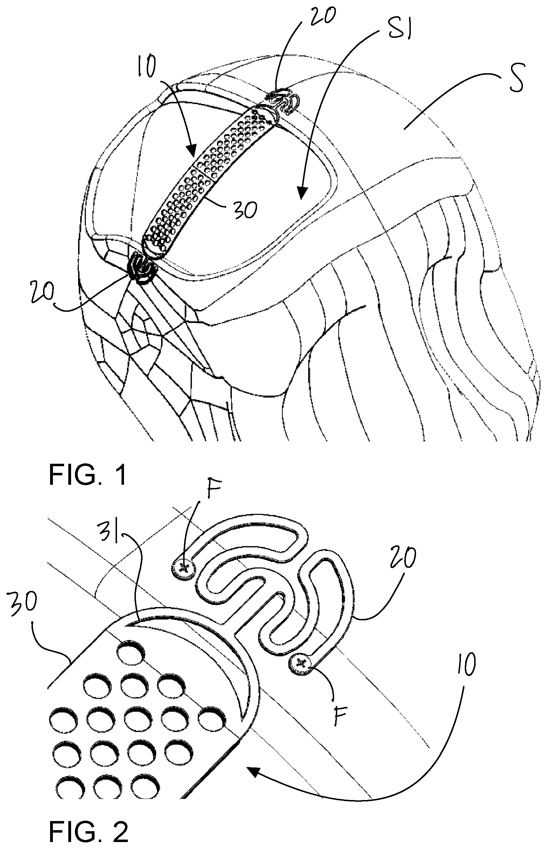

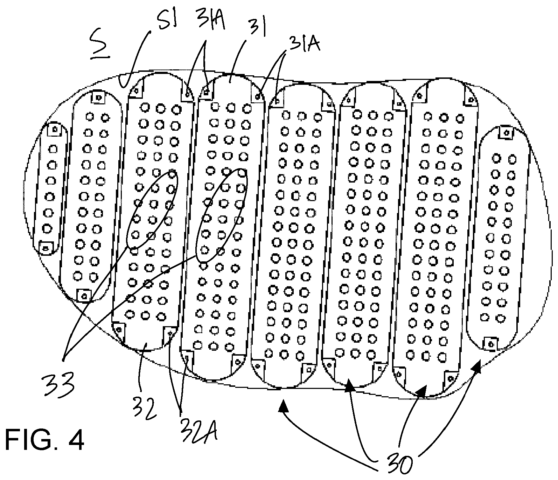

Referring to the drawings and more particularly to Fig. 1, there is illustrated a cranioplasty prosthesis in accordance with the present disclosure at 10. The cranioplasty prosthesis 10 is shown spanning across an opening S1 in a skull S. The opening S1 may result from a craniectomy procedure, for example. For simplicity, a single cranioplasty prosthesis 10 is shown and therefore only partially covers the opening S1 in the skull S but a plurality of the cranioplasty prosthesis 10 could be used in a side-by-side arrangement, in a manner similar to the embodiment shown in Fig. 4.

The cranioplasty prosthesis 10 is of the type that is used to temporarily or permanently cover the opening S1, for instance pursuant to a cranioectomy procedure or a craniotomy procedure. The expression "cranioplasty" is used as a moniker for the prosthesis 10 in that the prosthesis 10 serves to cover the opening S1 in the skull S. While the use of the cranioplasty prosthesis 10 is described in a context of craniotomy, craniectomy, or cranioplasty, it may be used in other circumstances as well.

The cranioplasty prosthesis 10 is shown as being made of a pair of attachment devices 20 at opposite ends of a prosthetic blade 30, in accordance with a variant of the present disclosure. The attachment devices 20 may be deformable to allow an adjustment of the prosthetic blade 30 relative to the skull S, while the prosthetic blade 30 defines the structural component of the cranioplasty prosthesis 10 that acts as a temporary or permanent skull shell portion to cover the opening S1. The prosthetic blade 30 may also be made of a shapeable material, such as a metallic plate, such that the prosthetic blade 30 may be shaped into a given curvature in continuity with the surrounding cranium surfaces, while providing suitable impact resistance to protect the brain. In the illustrated embodiment of Figs. 1 to 3, the attachment devices 20 and the prosthetic blade 30 are monoblock in construction. For example, the attachment devices 20 and the prosthetic blade 30 may be made of a single sheet of material that may be cut in any appropriate way, such as by laser cutting, CNC machining, casting, etc. It is also possible to mechanically attach the attachment devices 20 to the prosthetic blade 30 so as to have three components, separable from one another. For example, the attachment devices 20 may be secured to the prosthetic blade 30 by screws, by welding, by glueing, etc. The attachment devices 20 may also be used as fixations for bone flaps, i.e., with the prosthetic blade 30.

In accordance with a variant of the present disclosure, the cranioplasty prosthesis 10 may have a single one of the attachment device(s) 20, i.e., only at one end of the prosthetic blade 30, with the prosthetic blade 30 anchored directly to the skull at the other end. In accordance with another variant, the prosthetic blade 30 may be used without the attachment device 20, with other securement means provided to anchor the prosthetic blade 30 to the skull S, such as screws. Such prosthetic blade 30 may not allow a dynamic adjustment of shape at the coverage of the opening S1, for instance to address intracranial pressure concerns, but may nevertheless form a prosthetic flap that provides suitable structural integrity. Moreover, the prosthetic blade 30 may be shaped into a given geometry to match surrounding outer cranium surfaces, while also relieving some pressure due to their thinness in comparison to bone flaps.

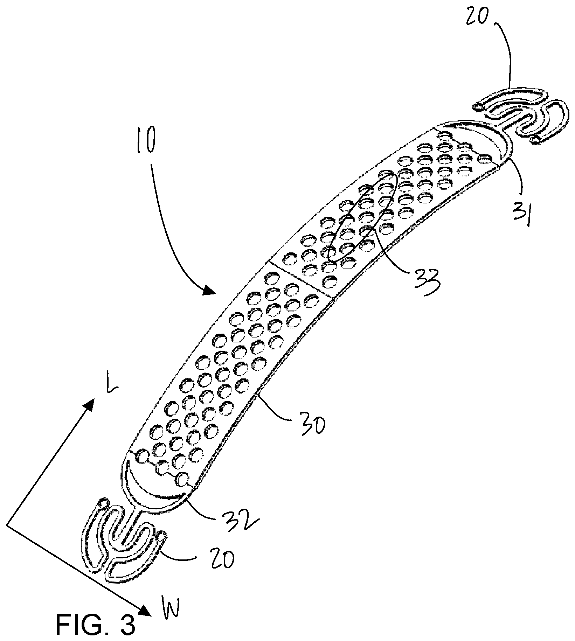

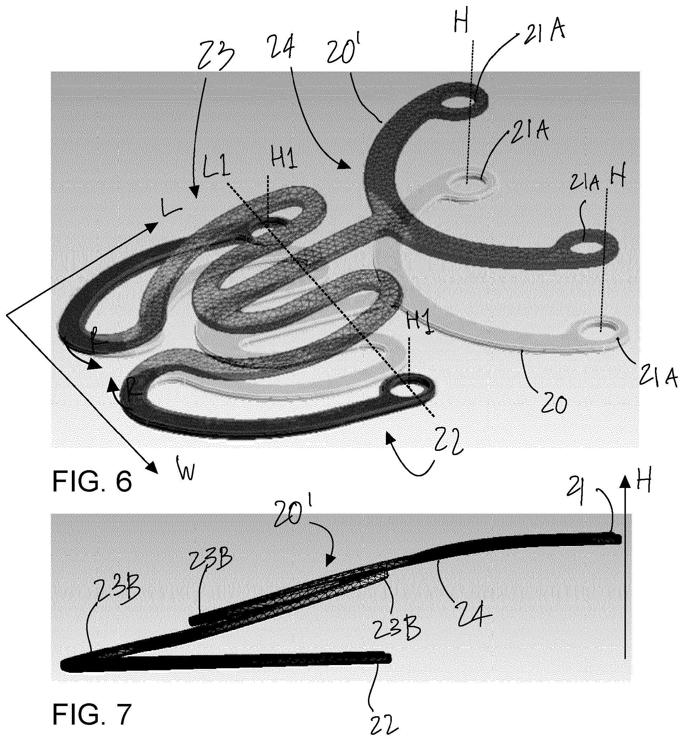

An enlarged view is provided in Fig. 2 to illustrate that the attachment device 20 may be secured to the bone of the skull by way of screws F. Other securement means are considered as well, including bolts, suture, adhesives, etc. Referring to Fig. 3, it is observed that the cranioplasty prosthesis 10 has a planar body extending in length L, relative to width W, with a thickness of the planar body being substantially thinner than the width W (e.g., at least 10 times less). The planar body may be made of a biocompatible material or of a combination of materials, such as a metal or a polymer. For example, titanium is well suited to be used to define the cranioplasty prosthesis 10, such as titanium in a sheet format. The material is a rigid material but may exhibit some flexibility due to its limited thickness. Therefore, the cranioplasty prosthesis 10 may have portions deformable out of a flat plane (e.g., when the cranioplasty prosthesis 10 is made from a sheet). The deformation may be in the elastic deformation range and may also reach plastic deformation. This may be observed in Fig. 3 in which it is shown that the prosthetic blade 30 is curved, for example to match a shape of surrounding outer cranium surfaces. Due to the use of a rigid material, and the width and length substantially greater than the thickness, in-plane deformation may not be possible, i.e., deformation while the cranioplasty prosthesis 10 remains planar. More specifically, the cranioplasty prosthesis 10 will not deform while remaining in the L-W plane (Fig. 6). The cranioplasty prosthesis 10 may also oppose to any buckling by its configuration.

The out-of-plane deformation of the prosthetic blade 30, embodied in Fig. 2 by the curve, may allow a user to manually define a shape of the prosthetic blade 30, for instance to emulate a geometry of the bone flap that is removed. In a variant, the shape may be obtained by applying the prosthetic blade 30 against a target site of the skull S, before craniectomy. Instruments may optionally be used to shape the blades 30. Measuring instruments, that may for example emulate the shape of the skull, may be used to obtain physical shape representations. Blades 30 of different length may be put side by side, to customize a shape of a flap constituted of numerous blades 30, as in Fig. 4. The elongated nature of the blades 30, whether or not part of the cranioplasty prosthesis 10, may facilitate their insertion in a small incision, for example with endoscopic maneuvers. A single incision, sized based on the width of a single blade 30, may be used for the insertion of all blades 30, when numerous blades are used. Moreover, the rounded ends of the blades 30, though optional, are without corners and hence reduce risks of catching surrounding soft tissue when the blades 30 are slid into position, for instance by a pushing movement on the trailing end of the blades 30 or of the cranioplasty prosthesis 10. Other tapering shapes are considered for the blades 30, in addition to the hemi-circular end geometry shown.

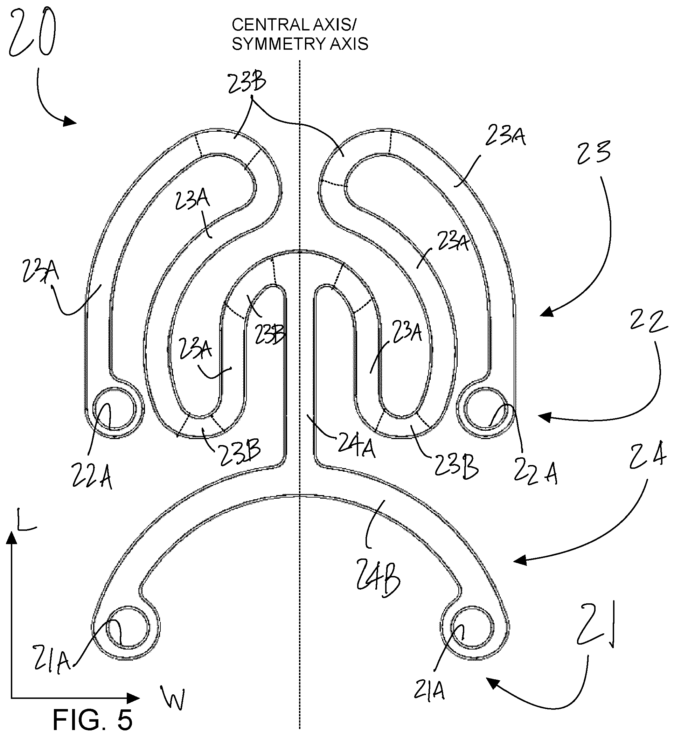

Referring to Fig. 5, an exemplary embodiment of the attachment device 20 is provided. As explained above, the attachment device 20 may or may not be part of the cranioplasty prosthesis 10. For example, the attachment device 20 may be used alone to join a skull to a bone flap, to a prosthetic shell, or to a prosthesis differing from the prosthetic blade 30. The attachment device 20 is designed to allow relative and constrained movement between bone flap, prosthetic shell, or prosthesis covering the opening S, and the skull surface surrounding the opening S. The movement may be described as being mostly out of plane, with a residual bi-directional movement in length L.

The attachment device 20 of Fig. 5 is shown having a planar body and may be made of a sheet material, in a monoblock construction. For example, the attachment device 20 of Fig. 5 may be made of a single sheet of material that may be cut in any appropriate way, such as by laser cutting, CNC machining, casting, etc. For reference, the attachment device 20 may, in a neutral position, lie in a plane defined by the length L and the width W. The neutral position may be a native condition of the attachment device 20. The plane defined by the length L and the width W may be a flat plane, but may also have a curved condition, i.e., a curved plane.

The attachment device 20 has a first connection end 21 and a second connection end 22. The first connection end 21 is defined by a pair of holes 21A that are configured to receive a fastener such as screw F of Fig. 2. Fewer or more holes 21A may be present, or other connection members may be present, such as spikes, tacks, nails, glue, etc. Likewise, the second connection end 22 also has a pair of holes 22A (or more or fewer) to receive fasteners such as screw F of Fig 2. In an embodiment, the holes 21A are aligned with one another along the width W. Likewise, in an embodiment, the holes 22A are aligned with one another along the width W. Other arrangements are considered. In a variant, the holes 21A and/or the holes 22A have the illustrated circular shape (e.g., straight hole, hole with counterbore, hole with countersink). Complementary fasteners such as screws F (Fig. 2) or the like (pins, bolts, etc) are received in the holes 21A and/or 22A, thereby defining a rotational joint, provided that the fasteners are not too tightly installed. The rotational joints may therefore enable a rotational movement, as described below and shown by R, about rotational axes H1 that may be generally parallel to height H.

The first connection end 21 and the second connection end 22 are interconnected by a coil portion 23, for instance formed of multiple coils, and by a frame portion 24. The coil portion 23 may also be referred to as a switchback mechanism, with multiple switchbacks. In use, the attachment device 20 has the first connection end 22 secured to the skull S adjacent to the skull opening S1. The second connection end 21 may be connected to a bone flap or prosthesis covering the skull opening S1, such as the prosthetic blade 30. The reverse arrangement is also possible. Therefore, while the second connection end 22 is shown as having holes to be screwed to a component, the second connection end 22 may be integrally connected to the blade portion 30 as shown in Fig. 1.

The coil portion 23 is responsible for allowing out-of-plane movement of the second connection end 22 relative to the first connection end 21, with Fig. 6 showing the attachment device at 20' after having sustained an out-of-plane deformation, relative to the attachment device 20 in its neutral position (also referred to herein as original condition). The coil portion 23 is configured to constrain movement of the first connection end 21 such that the first connection end 21 is practically superposed in height H with itself in a manner shown in Figs. 6 and 7. The rotational joints at the end 22 may contribute to this quasi-superposition. Stated differently, the coil portion 23 is arranged to limit the first connection end 21 to movement along the height H. While the first connection end 21 may also move along length L, the variation in distance along length L when projected onto the neutral plane (i.e., original condition) is substantially less than the variation in distance along height H. The frame portion 24, on the other end, is between the first connection end 21 and the coil portion 23. The frame portion 24 is a rigid component (i.e., no in-plane deformation) that may optionally be located over the kerf, to force a unidirectional deformation characteristic of the attachment device 20, namely to enable movement upwardly from a neutral condition, as explained below. Thus, movement of the first connection end 21 in the width W direction is limited or negligible, due to the physical constraints imposed by the frame portion 24. As can be observed, the coil portion 23 is connected to the frame portion 24, and the frame portion 24 enables various types of deformation of the coil portion 23, while the frame portion 24 may deform in flexion only, in a variant. Although the movement is described as being that of the first connection end 21, similar behavior may apply to the first connection end 21 from the perspective of the second connection end 22. In use, in an embodiment, it is the second connection end 22 that leads when slid into an incision. Although not necessary, the flaring shape from the leading end to the trailing holes 22A, and the trailing position of the holes 22A relative to a tip of the coil portion 23 in direction -L, limits movement in H, and contributes to maintaining the attachment device 20 planar and moving along the surface of the skull. The flaring shape from the leading end to the trailing holes 22A may be described as being arcuate, or may have other shapes, if present.

The coil portion 23 and frame portion 24 constrain the movements of the ends 21 and 22 relative to one another, by having various components. The coil portion 23 has struts 23A that extend at least partially in the length L direction. The struts 23A may be interconnected by webs 23B. The webs 23B may be shorter than the struts 23A, and may be located at ends of the struts 23A. For example, the webs 23B may be transverse to the struts 23A and may extend at least partially in the width W direction. Therefore, when one of the ends 21 and 22 is subjected to a force, such as that shown in Fig. 6, the webs 23B deform by the leveraging effect of the struts 23A, allowing the out-of-plane movement of the struts 23A. The webs 23B serves as rotational joints (along W), by deformation. Although the struts 23A and webs 23B are described as distinct features, the coil portion 23 may have curved sections that behave as struts 23A and webs 23B, the webs 23B being at junctions with other struts 23A to which the curved sections are connected. Again, the struts 23A and webs 23B may all be made of a single sheet material. In a variant, the webs 23B may be said to exhibit torsion when out-of-plane movement occurs for the struts 23A. In parallel to the torsion of the webs 23B, the struts 23A may exhibit flexion, in the out-of-plane movement. In a variant, any such torsion may be in elastic deformation (though it could also be in plastic deformation). When subjected to a deformation, the attachment device 20 may be defined as a compliant mechanism, i.e., a flexible body that elastically deforms. Moreover, by its configuration, the attachment device 20 may be said to be unidirectional in its displacement from its original condition (shown in lighter tone in Fig. 6), in that it may move only in one direction from the original condition (neutral plane), namely toward the deformed condition shown in Fig. 6. In its original condition, the attachment device 20 cannot move in the other direction through normal in-use forces and pressures. Stated differently, the attachment device 20 cannot deform in negative H from the neutral plane/original condition. This is because the frame portion 24 of the attachment device 20 is essentially transverse to and above a kerf between skull and flap, such that the attachment device 20 prevents inward movement of the flap. i.e., depression of the bone flap. The frame portion 24 is shown as having a strut 24A, at the first end of which the coil portion 23 is connected. The coil portion 23 may be said to be divided in two sets of coils from the strut 24A of frame portion 24. In a variant, the strut 24A extends along L. Other arrangements are possible, with pairs of struts 24A, etc. The frame portion 24 may further include a frame member 24B at the end of which are located the connection holes 21A of the first connection end 21. Therefore, there is no coil portion, thus deformation of the frame portion 24 is limited, in contrast to the coil portion 23. This can be observed in Figs. 6 and 7, for example.

Moreover, as observed from Fig. 6, the optional rotational joints at the end 22 (with fasteners such as F (Fig. 2)), enable rotation of the first coil portions in direction R as the attachment device 20 moves out of the neutral plane, i.e., rotation about axes H1. This may be possible by the fact that the connection holes 22A are each connected to respective coils of the coil portion 23, referred to as sets above. This rotation results in an expansion of the attachment device 20 along direction L, contributing to the footprint of the attachment device 20 in the deformed condition to be close to the footprint in the neutral condition. It can also be observed that the coil portion 23 has segments that are in a direction opposite the connection end 21, relative to line L1 passing through the connection holes 22A. This may result in a greater span of movement of the first connection end 21 relative to the second connection end 22.

In a variant, the second connection end 22, i.e., featuring the rotational joints, is on the skull, whereas the first connection end is connected to the flap or to the blade 30. The first connection end 21 has a single member interconnecting the holes 21A, and thus there may not be any substantial rotation at the holes 21A.

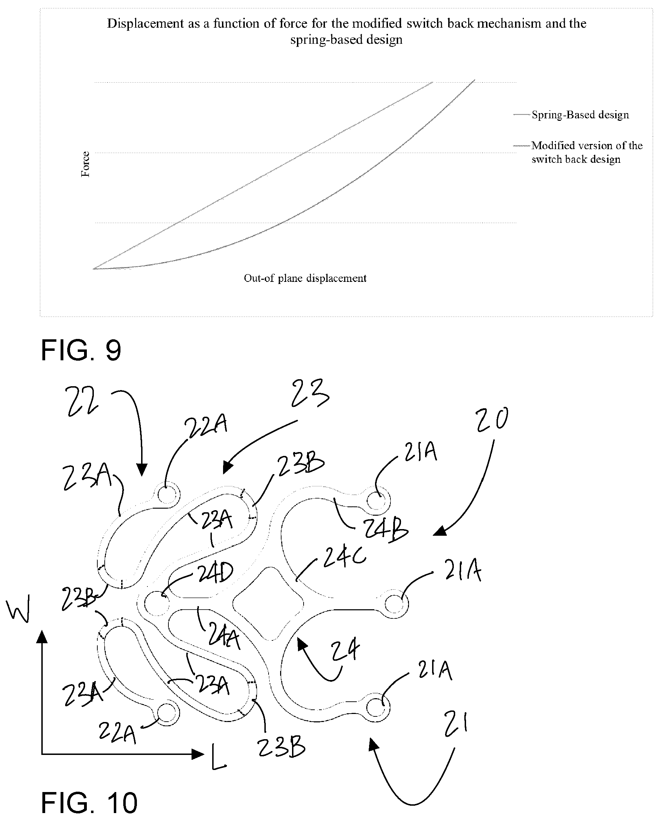

By the combination of the various actions, i.e., rotation, torsion, flexion, with deformations occurring in the elastic deformation range, the displacement versus force plot line may exhibit a non-linear behavior (in contrast to spring-based systems complying with Hooke's law), and thus may result in greater displacement to force ratios. This may be useful when relieving intracranial pressure. An exemplary graph is provided in Fig. 9 to illustrate this.

Referring to Fig. 10, another embodiment of the attachment device 20 is shown, and like reference numerals represent like elements for all embodiments shown herein. The attachment device 20 may include a closed frame 24D as part of the frame portion 24. The closed frame 24 is a rigid component (i.e., no in-plane deformation) that may be optionally located over the kerf, to force the unidirectional deformation characteristic of the attachment device 20. The presence of a third hole 21A may also contribute to the rigid securing of the first connection end 21 to a bone flap, for example. The presence of an anchor hole 24D at the junction of the coil portion 23 and the frame portion 24, or in the frame portion 24, may block rotation about H1, and could be present to give the attachment device 20 the possibility to be used as described above, or as a fixed anchor. The frame portion 24 (e.g., via the strut 24A and/or hole 24D) interconnects the two halves of coil portions 23, and thus blocks flexion, torsion, rotation. The two halves may not be similar, and may be regarded as two sets of coils, i.e., a first set of coils and a second set of coils, the first set of coils and the second set of coils connected to a respective one of the second connection holes 22A and merging.

Referring to Fig. 5, the attachment device 20 may have one or more axes of symmetry, with one such axis of symmetry being substantially parallel to the length L direction. By being connected on both sides of the axis of symmetry, or otherwise on both sides of a central axis parallel to length L, the attachment device 20 is generally prevented from a torsion along length L.

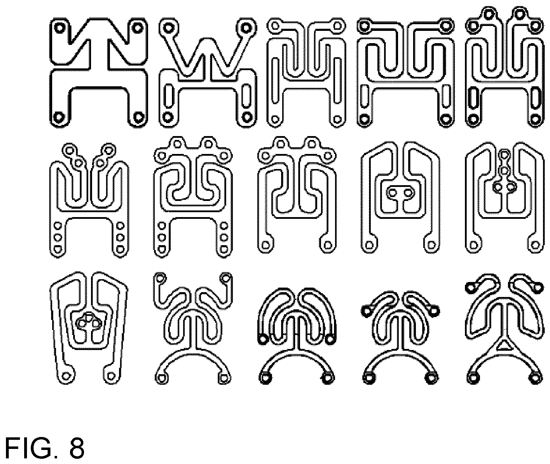

Fig. 8 shows different attachment device shapes that can be used to achieve such out-of-plane deformation. All of the embodiments of Fig. 8 may be made from a sheet of material with all of these figures being from a plane view. The shapes of Fig. 8 have sets of struts 23A and webs 23B in their coil portions 23, and a frame portion 24 that would be positioned above a kerf. Moreover, although it is not necessary, the attachment device shapes all have a symmetry axis that is parallel to the length L direction. In all of the attachment devices of Fig. 8, connection features, such as holes, are on opposite sides of a central axis that is parallel to the length L direction. The central axis may be coincident with the symmetry axis.

The proposed thickness of the attachment device 20 could vary between 0,4mm and 0,6mm, inclusively. It may be possible to make it thicker or thinner. This limited thickness is used in the interest of an appropriate aesthetic outcome. The width may be 25mm ± 2 mm and the length between 20mm and 35mm, inclusively. These dimensions could vary depending on the dynamic displacement needed, with these dimensions being merely given as an example. The material used is for example titanium grade 23 (TiAl6V4 ELI). This material may be used for its capacity to withstand significant deformation before plastic deformation. Chosen for its malleability and the possibility of manufacturing in its sheet form, the titanium grade 23 is well suited to be used for the attachment device 20 and for the prosthetic blade 30.

The attachment device 20 is well suited to anchor a prosthesis or bone flap to the skull and allow a dynamic unidirectional movement, i.e., movement essentially limited to displacement of a connection end 21 or 22 in the height H direction, from a neutral plane, and possibly back toward the neutral plane. Accordingly, the attachment device 20 allows displacement of a bone flap or prosthesis as a reaction to intracranial pressure. Consequently, the increase in the cerebral volume may limit damages caused by intracranial hypertension. In its neutral position, the attachment device 20 offers its maximum shearing resistance, thereby preventing an inward movement of the bone flap or prosthesis. The flaring shape from the leading end to the holes 22A may also contribute to the prevention of inward movement. The attachment device 20 may be made of any appropriate material that is semi-flexible, biocompatible and/or biodegradable, such as metals and plastics.

The attachment device 20 may be used in a hybrid manner, for instance by use as part of the cranioplasty prosthesis 10 or to connect a bone flap to a remainder of the skull. For example, in the latter scenario, while not as optimal in addressing intracranial pressure issues as with the cranioplasty prosthesis 10 (as the prosthetic blade 30 is substantially thinner than a bone flap), the presence of the attachment device 20 may allow some form of decompression as well.

Referring to Figs. 1 to 4, the prosthetic blade 30 is shown as having an elongated body between its first connection end 31 and its second connection end 32. The elongated body is a planar body and may also be made of a sheet material, in a monoblock in construction, that is manufactured for example using laser cutting, CNC machining, casting, etc. For reference, the prosthetic blade 30 may, in a neutral position, lie in the plane defined by the length L and the width W. The neutral position may be a native condition of the prosthetic blade 30. The plane defined by the length L and the width W may be a flat plane, but may also have a curved condition, i.e., a curved plane. The main surfaces of the prosthetic blade 30, i.e., one having the brain in use and one oriented away from the skull, extend in the length L and width W directions. A thickness of the prosthetic blade 30 may be substantially less than the length and width of the main surfaces, whereby the prosthetic blade 30 may be deformable out of plane from its native condition, in the elastic and optionally plastic deformation range.

So as not to damage surrounding tissue, the first connection end 31 and the second connection end 32 may be rounded or have like arched shapes, though other shapes are also possible. In the illustrated embodiments, the overall shape of the prosthetic blade 30 may be described as obround. As shown in Figs. 1 to 3, the prosthetic blade 30 may have the attachment devices 20 integral therewith, or may have the attachment devices 20 mechanically connected thereto, such as with fasteners. As observed in the variant of Fig. 4, holes 31A and 32A may be defined in the first connection end 31 and the second connection end 32, respectively, for the prosthetic blade 30 to be anchored directly to the skull by a fastener (e.g., screw F of Fig. 2), or to the attachment device 20 or other attachment means. The prosthetic blade 30 may be used alone, for instance in scenarios in which no expansion is required to counter any increase in intracranial pressure.

A plurality of holes 33 may be defined in a main surface of the prosthetic blade 30 so as to lessen the weight of the prosthetic blade 30, increase its flexibility and/or allow a scanning through the prosthetic blade 30, for instance if a radiopaque material is used for the prosthetic blade 30. The holes 33 may also serve for the attachment of devices to the blades 30, such as fillers, monitoring devices or sensors, drains, etc. As observed in Fig. 4, various lengths and widths of prosthetic blades 30 are possible. Therefore, during surgery, an operator may have access to a plurality of blades of different sizes, as shown in Fig. 4, in order to have customized coverage of the opening S1, with the blades 30 being used in the side-by-side arrangement shown, though other patterns are possible, with overlap, for example. In Fig. 4, it is observed that the prosthetic blades 30 do not extend onto the skull S, and this implies that attachment devices 20 or equivalents may be used. However, the prosthetic blades 30 could extend to overlap a surface of the skull S for the blades 30 to be secured directly to the skull S.

In Fig. 1, it is observed that the prosthetic blades 30 are positioned on a top surface of the skull S. Moreover, the prosthetic blades 30 may be thinner than that of the bone flap removed, due to the use of a sheet material or equivalent thin material. As a consequence, a clearance may be defined between the main surface of the prosthetic blade 30 facing the brain and soft tissue inside the skull S. This may result in an increase of the intracranial volume, and may have contribute to the reduction of the intracranial pressure. Therefore, while in some instances it may be required to use attachment devices 20 or equivalents that allow dynamic deformation based on local pressure, in some cases the use of the prosthetic blades 30 suffice in providing increased cranial volume to reduce intracranial pressure. The elongated shape of the prosthetic blades 30 may facilitate their insertion under the skin, for instance via 2-3 cm incisions, with or without endoscopic assistance.

The attachment device 20 may be used to treat several lesions resulting from traumas, subdural hematomas, sub-arachnoid hemorrhages, intracerebral hemorrhages, cerebral venous thrombosis, meningitis, empyema, osteomyelitis, hydrocephalus, tumors, or like intracranial disorders. With the cranioplasty prosthesis 10, attachment device 20 and/or prosthetic blade 30 of the present disclosure, it is possible to treat intracranial hypertension with a single surgery. In doing so, risk of complications may be considerably reduced and patient recuperation time may be lessened. Moreover, due to the single intervention, post-surgical complications may be avoided. The cranioplasty prosthesis 10 is a universal and dynamic bone flap prosthesis that may be implanted at the moment of craniotomy or craniectomy. Not only does the cranioplasty prosthesis 10 form an efficient protection against impacts and may cause a reduction in the intracranial pressure, the surgery costs related to the use of the cranioplasty prosthesis 10 may be reduced.

The attachment device 20 can generally be described as having a body defined from a sheet material and including: a first connection end having at least a first connection hole configured to cooperate with a fastener to anchor the attachment device to a bone flap or prosthesis covering at least part of the opening in the skull, a second connection end having at least a second connection hole configured to cooperate with a fastener to anchor the attachment device to a skull adjacent to an opening in the skull, a frame portion extending from the first connection end, and a coil portion between the frame portion and the second connection end, the coil portion having struts configured for deforming in flexion, and webs between the struts configured for deforming in torsion, wherein the frame portion is configured to be located over a periphery of the opening in the skull to block inward movement, and wherein the coil portion enables an out-of-plane deformation of the attachment device for the first connection end to move out of a neutral plane with the second connection end. It can be described in one embodiment as a pair of coils each connected to a bone by a screws or like fastener, forming a rotational joint, and a frame portion joining the pair of coils and connecting same to a blade or flap.

The prosthetic blade 30 may be described as being used for covering a skull opening in cranioplasty, and may have a body defined from a sheet material and including: a first connection end configured to be anchored to a first position of a skull, a second connection end configured to be anchored to a second position of the skull, the first position and the second position being separated by the skull opening, and an elongated plate portion between the first connection end and the second connection end, the elongated plate portion having a main surface facing toward the skull opening. The sheet material is a rigid biocompatible material deformable out of plane.

The above description is meant to be exemplary only, and one skilled in the art will recognize that changes may be made to the embodiments described without departing from the scope of the invention disclosed. Still other modifications which fall within the scope of the present invention will be apparent to those skilled in the art, in light of a review of this disclosure, and such modifications are intended to fall within the appended claims.

## Claims

1. An attachment device (20) for cranioplasty comprising
a body defined from a sheet material and including:
a first connection end (21) having at least a first connection hole (21A) configured to cooperate with a fastener (F) to anchor the attachment device (20) to a bone flap or prosthesis covering at least part of the opening in the skull,
a second connection end (22) having at least a second connection hole (22A) configured to cooperate with a fastener (F) to anchor the attachment device (20) to a skull adjacent to an opening in the skull, the body extending in a lengthwise direction (L) from the first connection end (21) to the second connection end (22),
a frame portion (24) extending from the first connection end (21), and
a coil portion (23) between the frame portion (24) and the second connection end (22), the coil portion (23) having struts (23A) configured for deforming in flexion, and webs (23B) between the struts (23A) configured for deforming in torsion, **characterised in that** the struts (23A) extend at least partially along the lengthwise direction (L), the webs (23B) extending in a widthwise direction (W)
wherein the coil portion (23) enables an out-of-plane deformation of the attachment device (20) for the first connection end (21) to move out of a neutral plane with the second connection end (22).

2. The attachment device (20) according to claim 1, wherein the second connection end (22) has a pair of the second connection holes (22A).

3. The attachment device (20) according to claim 2, wherein the coil portion (23) has a first set of coils and a second set of coils, the first set of coils and the second set of coils connected to a respective one of the second connection holes (22A) and merging at the frame portion (24).

4. The attachment device according to claim 3, wherein the second connection holes (22A) form rotational joints with fasteners (F) configured to secured the second connection end (22A) to the skull, the rotational joints enabling lengthwise expansion of the attachment device (20).

5. The attachment device (20) according to any one of claims 2 to 4, wherein a part of the coil portion (23) is on one side of a line passing through the second connection holes (22A), the first connection end (21) being on the other side of the line.

6. The attachment device (20) according to claim 5, wherein the part flares in toward the line.

7. The attachment device (20) according to any one of claims 1 to 6, wherein the frame portion (24) has a closed frame between the first connection end (21) and the coil portion (23).

8. The attachment device (20) according to any one of claims 1 to 7, wherein an entirety of the contour edges of the body is arcuate.

9. The attachment device (20) according to any one of claims 1 to 8, wherein the first connection end (21) has a pair of the first connection holes (21A).

10. The attachment device (20) according to any one of claims 1 to 9, wherein the frame portion (24) has a strut (24A) extending lengthwise from the coil portion (23).

11. The attachment device (20) according to any one of claims 1 to 10, wherein the attachment device (20) has a symmetry axis extending lengthwise.

12. The attachment device (20) according to any one of claims 1 to 11, wherein the frame portion (24) is configured to be located over a periphery of the opening in the skull transverse to a kerf to block inward movement.

13. A cranioplasty prosthesis (10) comprising:
a pair of the attachment device (20) according to any one of claims 1 to 12; and
a prosthetic blade (30) a body defined from a sheet material and including:
a first connection end (31) configured to be anchored to a first position of a skull by one of the attachment devices (20),
a second connection end (32) configured to be anchored to a second position of the skull by the other of the attachment devices (20), the first position and the second position being separated by a skull opening, and
an elongated plate portion between the first connection end (31) and the second connection end (32), the elongated plate portion having a main surface facing toward the skull opening,
wherein the sheet material is a rigid biocompatible material deformable out of plane.

14. The cranioplasty prosthesis (10) according to claim 13, wherein the body has holes (33).

## Patentansprüche

1. Befestigungsvorrichtung (20) für Kranioplastik, umfassend
einen Körper, welcher aus einem Bahnmaterial definiert ist und umfasst:
ein erstes Verbindungsende (21), welches wenigstens ein erstes Verbindungsloch (21A) aufweist, welches dazu eingerichtet ist, mit einem Befestigungselement (F) zusammenzuwirken, um die Befestigungsvorrichtung (20) an einem Knochendeckel oder einer Prothese zu verankern, welcher/welche wenigstens einen Teil der Öffnung in dem Schädel abdeckt,
ein zweites Verbindungsende (22), welches wenigstens ein zweites Verbindungsloch (22A) aufweist, welches dazu eingerichtet ist, mit einem Befestigungselement (F) zusammenzuwirken, um die Befestigungsvorrichtung (20) an einem Schädel angrenzend an eine Öffnung in dem Schädel zu verankern, wobei sich der Körper in einer Längsrichtung (L) von dem ersten Verbindungsende (21) zu dem zweiten Verbindungsende (22) erstreckt,
einen Rahmenabschnitt (24), welcher sich von dem ersten Verbindungsende (21) erstreckt, und
einen Windungsabschnitt (23) zwischen dem Rahmenabschnitt (24) und dem zweiten Verbindungsende (22), wobei der Windungsabschnitt (23) Streben (23A) aufweist, welche dazu eingerichtet sind, sich in Biegung zu verformen,
und Stege (23B) zwischen den Streben (23A), welche dazu eingerichtet sind, sich in Torsion zu verformen,
**dadurch gekennzeichnet, dass** sich die Streben (23A) wenigstens teilweise entlang der Längsrichtung (L) erstrecken, wobei sich die Stege (23B) in einer Breitenrichtung (W) erstrecken,
wobei der Windungsabschnitt (23) eine Verformung aus der Ebene heraus der Befestigungsvorrichtung (20) ermöglicht, sodass sich das erste Verbindungsende (21) aus einer Neutralebene mit dem zweiten Verbindungsende (22) herausbewegt.

2. Befestigungsvorrichtung (20) nach Anspruch 1, wobei das zweite Verbindungsende (22) ein Paar der zweiten Verbindungslöchern (22A) aufweist.

3. Befestigungsvorrichtung (20) nach Anspruch 2, wobei der Windungsabschnitt (23) ein erster Satz von Windungen und ein zweiter Satz von Windungen aufweist, wobei der erste Satz von Windungen und der zweite Satz von Windungen mit einem jeweiligen der zweiten Verbindungslöcher (22A) verbunden sind und an dem Rahmenabschnitt (24) zusammenlaufen.

4. Befestigungsvorrichtung nach Anspruch 3, wobei die zweiten Verbindungslöcher (22A) Drehgelenke mit Befestigungselementen (F) bilden, welche dazu eingerichtet sind, das zweite Verbindungsende (22A) an dem Schädel zu befestigen, wobei die Drehgelenke eine Längsausdehnung der Befestigungsvorrichtung (20) ermöglichen.

5. Befestigungsvorrichtung (20) nach einem der Ansprüche 2 bis 4, wobei ein Teil des Windungsabschnitts (23) an einer Seite einer Linie liegt, welche durch die zweiten Verbindungslöcher (22A) verläuft, wobei sich das erste Verbindungsende (21) an der anderen Seite der Linie befindet.

6. Befestigungsvorrichtung (20) nach Anspruch 5, wobei sich der Teil einwärts zu der Linie hin aufweitet.

7. Befestigungsvorrichtung (20) nach einem der Ansprüche 1 bis 6, wobei der Rahmenabschnitt (24) einen geschlossenen Rahmen zwischen dem ersten Verbindungsende (21) und dem Windungsabschnitt (23) aufweist.

8. Befestigungsvorrichtung (20) nach einem der Ansprüche 1 bis 7, wobei eine Gesamtheit der Konturränder des Körpers bogenförmig ist.

9. Befestigungsvorrichtung (20) nach einem der Ansprüche 1 bis 8, wobei das erste Verbindungsende (21) ein Paar der ersten Verbindungslöcher (21A) aufweist.

10. Befestigungsvorrichtung (20) nach einem der Ansprüche 1 bis 9, wobei der Rahmenabschnitt (24) eine Strebe (24A) aufweist, welche sich in Längsrichtung von dem Windungsabschnitt (23) erstreckt.

11. Befestigungsvorrichtung (20) nach einem der Ansprüche 1 bis 10, wobei die Befestigungsvorrichtung (20) eine Symmetrieachse aufweist, welche sich in Längsrichtung erstreckt.

12. Befestigungsvorrichtung (20) nach einem der Ansprüche 1 bis 11, wobei der Rahmenabschnitt (24) dazu eingerichtet ist, über einen Umfang der Öffnung in dem Schädel quer zu einem Schnittspalt positioniert zu sein, um eine Einwärtsbewegung zu blockieren.

13. Kranioplastik-Prothese (10), umfassend:
ein Paar der Befestigungsvorrichtungen (20) nach einem der Ansprüche 1 bis 12; und
ein Prothesenblatt (30), welches einen Körper umfasst, welcher aus einem Bahnmaterial definiert ist und umfasst:
ein erstes Verbindungsende (31), welches dazu eingerichtet ist, durch eine der Befestigungsvorrichtungen (20) an einer ersten Position eines Schädels verankert zu sein,
ein zweites Verbindungsende (32), welches dazu eingerichtet ist, durch die andere der Befestigungsvorrichtungen (20) an einer zweiten Position des Schädels verankert zu sein, wobei die erste Position und die zweite Position durch eine Schädelöffnung voneinander getrennt sind, und
einen länglichen Plattenabschnitt zwischen dem ersten Verbindungsende (31) und dem zweiten Verbindungsende (32), wobei der längliche Plattenabschnitt eine Hauptfläche aufweist, welche zu der Schädelöffnung hin gerichtet ist,
wobei das Bahnmaterial ein starres biokompatibles Material ist, welches aus der Ebene heraus verformbar ist.

14. Kranioplastik-Prothese (10) nach Anspruch 13, wobei der Körper Löcher (33) aufweist.

## Revendications

1. Dispositif de fixation (20) pour cranioplastie comprenant
un corps défini à partir d'un matériau en feuille et comportant :
une première extrémité de raccordement (21) présentant au moins un premier trou de raccordement (21A) configuré pour coopérer avec une attache (F) pour ancrer le dispositif de fixation (20) à un volet osseux ou à une prothèse recouvrant au moins une partie de l'ouverture dans le crâne,
une seconde extrémité de raccordement (22) présentant au moins un second orifice de raccordement (22A) configuré pour coopérer avec une attache (F) pour ancrer le dispositif de fixation (20) à un crâne adjacent à une ouverture dans le crâne, le corps s'étendant dans une direction longitudinale (L) de la première extrémité de raccordement (21) à la seconde extrémité de raccordement (22),
une portion de cadre (24) s'étendant à partir de la première extrémité de raccordement (21), et
une portion de bobine (23) entre la portion de cadre (24) et la seconde extrémité de raccordement (22), la portion de bobine (23) présentant des entretoises (23A) configurées pour se déformer en flexion, et des bandes (23B) entre les entretoises (23A) configurées pour se déformer en torsion, **caractérisé en ce que**
les entretoises (23A) s'étendent au moins partiellement le long de la direction longitudinale (L), les bandes (23B) s'étendant dans une direction transversale (W) dans lequel la portion de bobine (23) permet une déformation hors du plan du dispositif de fixation (20) pour que la première extrémité de raccordement (21) se déplace hors d'un plan neutre avec la seconde extrémité de raccordement (22).

2. Dispositif de fixation (20) selon la revendication 1, dans lequel la seconde extrémité de raccordement (22) présente une paire des seconds trous de raccordement (22A).

3. Dispositif de fixation (20) selon la revendication 2, dans lequel la portion de bobine (23) présente un premier ensemble de bobines et un second ensemble de bobines, le premier ensemble de bobines et le second ensemble de bobines étant raccordés à un respectif des seconds trous de raccordement (22A) et fusionnant au niveau de la portion de cadre (24).

4. Dispositif de fixation selon la revendication 3, dans lequel les seconds trous de raccordement (22A) forment des articulations rotatives avec des attaches (F) configurées pour attacher la seconde extrémité de raccordement (22A) au crâne, les articulations rotatives permettant l'expansion longitudinale du dispositif de fixation (20).

5. Dispositif de fixation (20) selon l'une quelconque des revendications 2 à 4, dans lequel une partie de la portion de bobine (23) est d'un côté d'une ligne passant à travers les seconds trous de raccordement (22A), la première extrémité de raccordement (21) étant de l'autre côté de la ligne.

6. Dispositif de fixation (20) selon la revendication 5, dans lequel la pièce s'évase vers la ligne.

7. Dispositif de fixation (20) selon l'une quelconque des revendications 1 à 6, dans lequel la portion de cadre (24) présente un cadre fermé entre la première extrémité de raccordement (21) et la portion de bobine (23).

8. Dispositif de fixation (20) selon l'une quelconque des revendications 1 à 7, dans lequel une totalité des bords de contour du corps est arquée.

9. Dispositif de fixation (20) selon l'une quelconque des revendications 1 à 8, dans lequel la première extrémité de raccordement (21) présente une paire des premiers trous de raccordement (21A).

10. Dispositif de fixation (20) selon l'une quelconque des revendications 1 à 9, dans lequel la portion de cadre (24) présente une entretoise (24A) s'étendant longitudinalement à partir de la portion de bobine (23).

11. Dispositif de fixation (20) selon l'une quelconque des revendications 1 à 10, dans lequel le dispositif de fixation (20) présente un axe de symétrie s'étendant longitudinalement.

12. Dispositif de fixation (20) selon l'une quelconque des revendications 1 à 11, dans lequel la portion de cadre (24) est configurée pour être située sur une périphérie de l'ouverture dans le crâne transversale à une encoche pour bloquer le mouvement vers l'intérieur.

13. Prothèse de cranioplastie (10) comprenant :
une paire du dispositif de fixation (20) selon l'une quelconque des revendications 1 à 12 ; et
une lame prothétique (30) un corps défini à partir d'un matériau de feuille et comportant :
une première extrémité de raccordement (31) configurée pour être ancrée à une première position d'un crâne par l'un des dispositifs de fixation (20),
une seconde extrémité de raccordement (32) configurée pour être ancrée à une seconde position du crâne par l'autre des dispositifs de fixation (20), la première position et la seconde position étant séparées par une ouverture crânienne, et
une portion de plaque allongée entre la première extrémité de raccordement (31) et la seconde extrémité de raccordement (32), la portion de plaque allongée présentant une surface principale orientée vers l'ouverture crânienne,
dans laquelle le matériau de feuille est un matériau biocompatible rigide déformable hors du plan.

14. Prothèse de cranioplastie (10) selon la revendication 13, dans laquelle le corps présente des trous (33).
